# EUROPÄISCHE PATENTSCHRIFT

(11) **EP 1 102 531 B1**
(45) Veröffentlichungstag und Bekanntmachung des Hinweises auf die Patenterteilung: **16.02.2005**
(21) Anmeldenummer: 00938755.6
(22) Anmeldetag: 06.06.2000
(51) Int. Cl.: A01K 67/033

(54) **VERFAHREN UND VORRICHTUNG ZUR AUFZUCHT VON FLIEGENLARVEN FÜR DIE THERAPEUTISCHE APPLIKATION**
METHOD AND DEVICE FOR REARING FLY LARVAE FOR THERAPEUTIC APPLICATION
PROCEDE ET DISPOSITIF DE CULTURE DE LARVES DE MOUCHES AUX FINS D'APPLICATION THERAPEUTIQUE

(30) Priorität: 08.06.1999 DE 19925996
(43) Veröffentlichungstag der Anmeldung: 30.05.2001
(73) Patentinhaber: Fleischmann, Wilhelm, Dr. med., 74321 Bietigheim-Bissingen (DE)
(72) Erfinder: Fleischmann, Wilhelm, Dr. med., 74321 Bietigheim-Bissingen (DE)
(74) Vertreter: Patentanwälte Westphal, Mussgnug & Partner
(86) Internationale Anmeldenummer: PCT/EP2000/005132
(87) Internationale Veröffentlichungsnummer: WO 2000/074478

(56) Entgegenhaltungen:
- WO-A-92/11760
- WO-A-95/26633
- CN-A- 1 213 497
- GB-A- 1 594 721
- DATABASE EPODOC [Online] EUROPEAN PATENT OFFICE, THE HAGUE, NL; CN-A-1142887, 19. Februar 1997 (1997-02-19) WUHAN SHENCHONGZHENQI HEALTH: XP002147657

## Beschreibung

Die Erfindung betrifft ein Verfahren und eine Vorrichtung zur Aufzucht von Fliegenlarven (Maden)für die therapeutische Applikation.

Zur Behandlung von Wundinfektionen und Wunden, die abgestorbenes Gewebe enthalten, z. B. zur Behandlung der diabetischen Gangrän, werden Fliegenlarven, sogenannte Maden eingesetzt, insbesondere Larven von Dipteren aus den Familien der Muscidae, Sarcophaginae und Calliphoridae (zum Beispiel Lucilia, Schmeißfliege). Die Maden werden für eine gewisse Zeit, z. B. etwa drei Tage, in die schwer therapierbare Wunde eingesetzt. Es hat sich gezeigt, dass die Maden in dieser Zeit abgestorbenes Gewebe in der Wunde entfernen, bakterielle Entzündungen beseitigen und die Wundheilung stimulieren. Diese Wirkung wird insbesondere durch das von den Maden abgesonderte Verdauungssekret hervorgerufen. Durch dieses Sekret wird abgestorbenes Gewebeverflüssigt, so dass es von den Maden als Nahrung aufgenommen werden kann. Das Sekret hat zusätzlich eine stark bakterizide Wirkung und fördert die Wundheilung.

Bei dieser Behandlungsmethode ist es notwendig, lebende Maden zur Verfügung zu haben, die in die Wunde appliziert werden und das heilsame Sekret absondern. Dadurch ergibt sich für die Anwendung dieser Methode ein erhebliches logistisches Problem.
Da die zeitliche Dauer des aktiven Madenstadiums, in welchem das heilsame Sekret abgesondert wird, relativ kurz ist und nur einige wenige Tage dauert, ist es notwendig, die Maden unmittelbar vor der Applikation von dem Hersteller, bei welchem die Maden gezüchtet werden, zu dem Anwender zu transportieren. Dies macht eine exakte zeitliche Planung notwendig. Außerdem sind die Maden relativ empfindlich und müssen während des Transports mit Luft und Nahrungsstoffen versorgt werden. Aufgrund dieser Schwierigkeiten ist die Fliegenlarven-Therapie in ihren Einsatzmöglichkeiten begrenzt und kann sich nicht in der wünschenswerten Weise durchsetzen.

Aus der WO 95/26633 ist es bekannt, Fliegenlarven zu züchten, die als Futtermittel verwendet werden. Fliegen werden in einem Behälter gehalten und legen ihre Eier auf ein Substrat ab. Die Eier werden gesammelt und gekühlt gelagert und dann in einen Brutbehälter gebracht, in welchem die Weiterentwicklung der Eier zum Madenstadium begünstigende Brutbedingungen bestehen.

Aus der CN 1 142 887 A und der CN 1 213 497 A ist ein Verfahren zur Züchtung von Fliegenlarven bekannt, bei welchem die Entwicklung der Eier bis zum Madenstadium in einem Brutbehälter unter Brutbedingungen abläuft.

Der Erfindung liegt die Aufgabe zugrunde, ein Verfahren und eine Vorrichtung zur Verfügung zu stellen, durch welche die therapeutische Applikation des Fliegenlarven-Sekrets vereinfacht und erleichtert wird.

Diese Aufgabe wird erfindungsgemäß gelöst durch ein Verfahren mit den Merkmalen des Anspruchs 1 sowie durch eine Vorrichtung mit den Merkmalen des Anspruchs 7.

Vorteilhafte Ausführungen der Erfindung sind in den rückbezogenen Unteransprüchen angegeben.

Die Erfindung nutzt die Kenntnis, die Aufzucht der Insekten in einem abgeschlossenen Biotop unter exakt definierten Umgebungsbedingungen durchzuführen, durch welche der Entwicklungszyklus gezielt beeinflusst werden kann. Hierdurch ist es möglich, die Entwicklung und das Verhalten der Insekten unter gezielt geänderten physikalischen, chemischen, biochemischen und biologischen Bedingungen zu studieren und für die medizinischtherapeutische und pharmazeutische Nutzung zu beeinflussen. Hierbei kann der zeitliche Ablauf des Entwicklungszyklus durch Änderung der Bedingungen gesteuert, das heißt verzögert oder .beschleunigt werden. Außerdem können durch gezielte Einflussnahme die Qualität und die Ausbeute gesteigert werden.

Bei der Anwendung des Verfahrens zur Gewinnung des Sekrets von Fliegenlarven für die therapeutische Applikation besteht ein wichtiger Aspekt darin, die Entwicklung der Fliegenlarven im Eistadium zu stoppen und die Eier unter entwicklungshemmenden Bedingungen zu lagern und/oder zu transportieren. Bei dem Hersteller, der die Fliegen züchtet, werden die Fliegeneier separiert und vorzugsweise desinfiziert, so dass sie ausreichend steril sind. Die sterilen Eier werden in einem abgeschlossenen Biotop, zum Beispiel einem Behälter eingeschlossen, in welchem die Weiterentwicklung der Eier hemmende Bedingungen geschaffen oder aufrechterhalten werden. Solche Bedingungen können darin bestehen, dass die Eier gekühlt werden und/oder in einer entfeuchteten Atmosphäre gelagert werden. Weiter können die Eier in einer sauerstoffarmen Atmosphäre gelagert werden, indem der Behälter beispielsweise evakuiert wird oder mit einem Inertgas gefüllt wird. Es sind auch reversible chemische/biochemische Einwirkungen (zum Beispiel Neb-TMOF)möglich, die für eine definierte Zeitspanne zur Oostase führen. Diese Maßnahmen können einzeln oder in Kombination angewendet werden. Unter diesen entwicklungshemmenden Bedingungen können die widerstandfähigen Eier über eine längere Zeitspanne lebensfähig gehalten werden.

Dies ermöglicht es, die Eier bei dem Hersteller über eine gewisse Zeitspanne auf Lager zu halten. Weiter können die Eier unter diesen Bedingungen in einfacher Weise zu dem Anwender transportiert werden, ohne 'Schaden zu nehmen. Auch bei dem Anwender, z.B. in einer Klinik kann ein gewisser Vorrat der Eier über eine gewisse Zeitspanne gelagert werden, so dass der jeweils aktuelle Bedarf gedeckt werden kann.

Soll das Madensekret appliziert werden, so wird bei dem Anwender die Weiterentwicklung der Eier in das Larvenstadium wieder in Gang gesetzt. Hierzu werden die entwicklungshemmenden Bedingungen beseitigt und die Eier werden Brutbedingungen ausgesetzt, so dass sie sich weiterentwickeln und innerhalb kurzer Zeit die Maden schlüpfen. Da die Eier beim Hersteller steril gemacht sind, sind auch die schlüpfenden Maden steril und können problemlos eingesetzt werden.

Es ist auch möglich, den künstlich geschaffenen, mikrobiologisch abgeschlossenen Lebensraum gezielt mit Mikroorganismen zu besiedeln, um die Wirksamkeit der Maden und ihres mikrobiologisch modifizierten Sekrets zu erhöhen oder zu verändern.

Die Maden werden in einem Applikationsbehälter in die Wunde eingebracht. Ein solcher Applikationsbehälter weist eine flüssigkeitsdurchlässige Wandung auf, die jedoch für die in dem Applikationsbehälter eingeschlossenen Maden undurchlässig ist. Beispielsweise kann der Applikationsbehälter ein flexibler Beutel mit einer netzartigen Wandung sein. Das von den Maden abgesonderte Sekret kann durch die Wandung des Applikationsbehälters hindurchtreten und in die Wunde gelangen. Das durch das Sekret aufgelöste Wundgewebe kann ebenfalls durch die Wandung des Applikationsbehälters hindurchtreten und von den Maden aufgenommen werden. Ein solcher Applikationsbehälter hat den Vorteil, dass die Maden und damit das von diesen abgesonderte Sekret gezielter lokalisiert appliziert werden können. Außerdem können die Maden zusammen mit dem Applikationsbehälter in einfacher Weise wieder aus der Wunde entfernt werden.

Das Bebrüten der Eier bis zum Schlüpfen der Maden erfolgt in einem Brutbehälter. In diesen Brutbehälter werden die Eier eingebracht und bei einer Temperatur zwischen 20 und 40° C bebrütet. Der Brutbehälter ist mit ausreichend Nährsubstanzen und Luft für die schlüpfenden Larven versehen, so dass diese sich bis zu dem Larvenstadium entwickeln können, in welchem sie das therapeutisch wirkende Sekret absondern. Dieser Brutbehälter ist vorzugsweise als Beutel aus einer Kunststofffolie hergestellt und enthält gegebenenfalls bereits ein saugfähiges Material zur Aufnahme der produzierten Wirksubstanzen unter sterilen oder mikrobiologisch kontrollierten Bedingungen.

Die Eier werden in dem Applikationsbehälter eingeschlossen in den Brutbehälter eingesetzt. Die kleinen Eier werden durch den Brutbehälter in dem Applikationsbehälter gehalten, so dass sie nicht durch dessen netzartige Wandung herausfallen. Wenn die Larven aus den Eiern schlüpfen, können sie die Nährstoffe aus dem Brutbehälter aufnehmen und wachsen in dem Applikationsbehälter zu der Grösse heran, bei der sie das therapeutisch wirkende Sekret absondern. Bei dieser Grösse können sie nicht mehr durch die netzförmige Wand des Applikationsbehälters hindurchschlüpfen. Der Applikationsbehälter kann nun aus dem Brutbehälter entnommen bzw. der Brutbehälter von dem Applikationsbehälter entfernt werden, so dass die Maden mit dem Applikationsbehälter direkt in die Wunde eingebracht oder mit einer porösen Wundeinlage in Verbindung gebracht werden können.

In einer für den Anwender besonders einfachen Ausführung werden die Eier bereits beim Hersteller in die Brutbehälter eingebracht und in den Brutbehältern zunächst für die Lagerung und den Transport unter entwicklungshemmenden Bedingungen gehalten. Die Brutbehälter werden hierzu evakuiert oder mit einem entfeuchteten Inertgas gefüllt oder chemisch/biochemisch oostatisch manipuliert. Ebenso oder gegebenenfalls zusätzlich können die Brutbehälter für die Lagerung und den Transport in einen Kühlbehälter eingebracht und gekühlt werden.

Sollen die Maden appliziert werden, so werden in den Brutbehältern Brutbedingungen geschaffen. Hierzu werden die Brutbehälter mit den Eiern in eine warme Umgebung gebracht. Den Brutbehältern werden sauerstoffhaltige Luft, die für die schlüpfenden Larven notwendige Nährlösung und gegebenenfalls aktivierende chemische oder biochemische Faktoren (zum Beispiel Peptidasen)zugeführt. Zum Zuführen von Sauerstoff kann die Wandung des Brutbehälters mit einer Kanüle punktiert werden oder einen Port enthalten. Gegebenenfalls können auf diese Weise auch die notwendige Nährlösung und gegebenenfalls die die oostatischen Einflüsse deaktivierenden Substanzen in den Brutbehälter eingebracht werden. Eine für den Anwender besonders bequeme Möglichkeit besteht darin, Luft, Nährlösung und sonstige Substanzen bereits herstellerseitig innerhalb des Brutbehälters in geschlossenen Beuteln oder geschlossenen Ampullen unterzubringen, die dann von dem Anwender zerstört werden, damit Luft und Nährlösung, sowie gegebenenfalls chemische, biochemische und mikrobiologische Komponenten frei in den Brutbeuteln austreten, das Schlüpfen fördern und von den geschlüpften Maden aufgenommen werden können.

Werden die Fliegen im Eistadium zu dem Anwender transportiert, so dass die Maden für die Applikation des Sekrets bei dem Anwender schlüpfen, so werden die Maden in der Regel vernichtet, sobald sie aus der Wunde entfernt sind oder das Sekret auf die Wundeinlage abgesondert haben.

Beim Hersteller wird vorzugsweise mit einem Stammbestandteil der Insekten der vollständige Entwicklungszyklus in dem zeitlich modifizierbaren und kontrollierbaren Biotop durchgeführt. Nach dem Schlüpfen der Maden werden diese gegebenenfalls zur Gewinnung des Sekrets genutzt, welches dann in applizierbarer Form für den Anwender zur Verfügung gestellt wird oder zur Herstellung von pharmazeutischen Präparaten verwendet werden kann. Am Ende des Larvenstadiums werden in dem Biotop dann die für die Verpuppung notwendigen Umgebungsbedingungen hergestellt, zum Beispiel trockene Luftverhältnisse. Die Maden verpuppen sich dann, so dass eine neue Generation von Insekten schlüpft, die wiederum zur Eiablage unter sterilen Bedingungen genutzt wird. Die Aufzucht der Insekten vom Ei bis zur geschlüpften Fliege unter zeitlich steuerbaren und kontrollierbaren Bedingungen gibt dem Hersteller die Möglichkeit, die Produktion der Maden bzw. des Madensekrets zeitlich und mengenmäßig der Marktnachfrage anzupassen. Außerdem können die Insektenstämme züchterisch beeinflusst werden, um eine Produktions- und Qualitätssteigerung zu erzielen. Schließlich ist eine gezielte mikrobiologische Einflussnahme auf das abgeschlossene Entwicklungsbiotop möglich, so dass ein therapeutischer Synergieeffekt von zum Beispiel gezielt zugegebenen Bakterien und dem von den Maden abgesonderten Sekret bewirkt werden kann.

## Patentansprüche

1. Verfahren zur Aufzucht von Fliegenlarven (Maden), bei welchem die Entwicklung der Fliegen zumindest vom Eistadium bis zum Madenstadium in einem abgeschlossenen Biotop abläuft, wobei durch Erzeugung, Aufrechterhaltung und Beseitigung entwicklungshemmender Bedingungen in dem Biotop die zeitliche Dauer des Eistadiums gesteuert wird,
**dadurch gekennzeichnet, dass**
für die therapeutische Applikation des Sekrets der Fliegenlarven die Eier in einem für das Sekret durchlässigen, für die geschlüpften Maden jedoch undurchlässigen Applikationsbehälter eingesetzt sind, während sie den Brutbedingungen ausgesetzt werden.

2. Verfahren nach Anspruch 1,
**dadurch gekennzeichnet, dass**
die zeitliche Dauer des Eistadiums für die Lagerung und/oder den Transport aufrechterhalten wird.

3. Verfahren nach Anspruch 2,
**dadurch gekennzeichnet, dass**
die Eier unter den entwicklungshemmenden Bedingungen zu dem Anwender transportiert werden und zur Vorbereitung der Applikation bei dem Anwender die entwicklungshemmenden Bedingungen beseitigt und Brutbedingungen hergestellt werden, so dass die Maden schlüpfen und sich zu dem das Sekret für die Applikation absondernden Madenstadium entwickeln.

4. Verfahren nach einem der vorhergehenden Ansprüche,
**dadurch gekennzeichnet, dass**
die Eier steril in dem Biotop eingeschlossen werden.

5. Verfahren nach Anspruch 1 oder 2,
**dadurch gekennzeichnet, dass**
die die Weiterentwicklung der Eier hemmenden Bedingungen ein Kühlen und/oder ein Entfeuchten und/oder ein Evakuieren und/oder eine Inertgasatmosphäre und/oder chemische oder biochemische Hemmstoffe einzeln oder in Kombination umfassen.

6. Verfahren nach einem der vorhergehenden Ansprüche,
**dadurch gekennzeichnet, dass**
die Eier in einem Brutbehälter den Brutbedingungen ausgesetzt werden, wobei die Eier in dem Applikationsbehälter in den Brutbehälter eingeschlossen sind.

7. Vorrichtung für die Aufzucht von Fliegenlarven (Maden) mit einem Behälter zur Aufnahme der Eier der Fliegen unter die Weiterentwicklung der Eier hemmenden Bedingungen und einem Brutbehälter, in welchem Brutbedingungen für die Eier aufrechterhalten oder erzeugt werden,
**dadurch gekennzeichnet, dass**
zur Gewinnung des Sekrets der Fliegenlarven für die therapeutische Applikation ein Applikationsbehälter vorgesehen ist, in welchen die Eier eingesetzt werden und in welchem die Eier unter Brutbedingungen gehalten werden, wobei die Wandung des Applikationsbehälters für Flüssigkeiten durchlässig, für die geschlüpften Maden jedoch undurchlässig ist.

8. Vorrichtung nach Anspruch 7,
**dadurch gekennzeichnet, dass**
der Applikationsbehälter eine flexible netzartige Wandung aufweist.

9. vorrichtung nach Anspruch 8,
**dadurch gekennzeichnet, dass**
der Applikationsbehälter in dem Brutbehälter eingeschlossen ist.

10. Vorrichtung nach einem der Ansprüche 7 bis 9,
**dadurch gekennzeichnet, dass**
der Brutbehälter ein Kunststofffolienbeutel ist.

11. Vorrichtung nach einem der Ansprüche 7 bis 10,
**dadurch gekennzeichnet, dass**
ein oder mehrere Brutbehälter mit in einen Applikationsbehälter eingeschlossenen Eier in dem Behälter aufgenommen sind, in welchem die entwicklungshemmenden Bedingungen aufrechterhalten sind, und dass in den Brutbehältern zunächst die entwicklungshemmenden Bedingungen herstellbar und aufrechterhaltbar sind und bei Bedarf in Brutund Ernährungsbedingungen umwandelbar sind.

12. Vorrichtung nach einem der Ansprüche 7 bis 11,
**dadurch gekennzeichnet, dass**
Luft und/oder Nährlösung und/oder chemische/biochemische/mikrobiologische Komponenten durch die Wandung des Brutbehälters, insbesondere mittels einer Kanüle zuführbar sind.

13. Vorrichtung nach einem der Ansprüche 7 bis 11,
**dadurch gekennzeichnet, dass**
Luft und/oder Nährlösung und/oder chemische/biochemische/mikrobiologische Komponenten in geschlossenen von außen zerstörbaren Vorratsbehältern in dem Brutbehälter eingeschlossen oder an ihm angebracht sind.

## Claims

1. Process for rearing fly larvae (maggots), in which the development of the flies at least from the egg stage to the maggot stage takes place in a closed biotope, wherein the time period of the egg stage is controlled by establishing, maintaining and removing development-inhibiting conditions in the biotope, **characterized in that**, for the therapeutic application of the secretion of the fly larvae, the eggs are placed in an application container while they are exposed to the breeding conditions, said application container being permeable to the secretion but impermeable to the hatched maggots.

2. Process according to Claim 1, **characterized in that** the time period of the egg stage is maintained for storage and/or transport.

3. Process according to Claim 2, **characterized in that** the eggs are transported to the user under the development-inhibiting conditions and, in order to prepare for application on the user, the development-inhibiting conditions are removed and breeding conditions are established, so that the maggots hatch and develop to the maggot stage at which they secrete the secretion for application.

4. Process according to one of the preceding claims, **characterized in that** the eggs are enclosed in the biotope under sterile conditions.

5. Process according to Claim 1 or 2, **characterized in that** the conditions which inhibit further development of the eggs comprise cooling and/or dehumidification and/or evacuation and/or an inert gas atmosphere and/or chemical or biochemical inhibitors, individually or in combination.

6. Process according to one of the preceding claims, **characterized in that** the eggs are exposed to the breeding conditions in a breeding container, wherein the eggs are enclosed in the application container inside the breeding container.

7. Device for rearing fly larvae (maggots), comprising a container for receiving the eggs of the flies under conditions which inhibit further development of the eggs and a breeding container in which breeding conditions for the eggs are maintained or established, **characterized in that**, in order to obtain the secretion of the fly larvae for therapeutic application, an application container is provided in which the eggs are placed and in which the eggs are kept under breeding conditions, wherein the wall of the application container is permeable to liquids but impermeable to the hatched maggots.

8. Device according to Claim 7, **characterized in that** the application container has a flexible, mesh-type wall.

9. Device according to Claim 8, **characterized in that** the application container is enclosed in the breeding container.

10. Device according to one of Claims 7 to 9, **characterized in that** the breeding container is a plastic film bag.

11. Device according to one of Claims 7 to 10, **characterized in that** one or more breeding containers comprising eggs enclosed in an application container are received in the container in which the development-inhibiting conditions are maintained, and **in that** in the breeding containers firstly the development-inhibiting conditions may be established and maintained and if necessary can be transformed into breeding and nutritive conditions.

12. Device according to one of Claims 7 to 11, **characterized in that** air and/or nutrient solution and/or chemical/biochemical/microbiological components can be supplied through the wall of the breeding container, in particular by means of a tube.

13. Device according to one of Claims 7 to 11, **characterized in that** air and/or nutrient solution and/or chemical/biochemical/microbiological components are enclosed in or applied to the breeding container in closed supply containers which can be destroyed from outside.

## Revendications

1. Procédé d'élevage de larves de mouches (asticots), selon lequel le développement des mouches se déroule au moins du stade de l'incubation jusqu'au stade larvaire dans un biotope fermé, en régulant la durée du stade d'incubation par la création, le maintien et la suppression de conditions empêchant le développement permettant de contrôler dans le biotope,
**caractérisé en ce que**
pour l'application thérapeutique des sécrétions des larves de mouches, les oeufs sont placés dans des récipients d'application perméables pour les sécrétions, mais imperméables pour les larves écloses, tandis qu'ils sont soumis aux conditions d'incubation.

2. Procédé selon la revendication 1,
**caractérisé en ce que**
la durée du stade d'incubation est maintenue pour l'entreposage et/ou le transport.

3. Procédé selon la revendication 2,
**caractérisé en ce que**
les oeufs sont transportés chez l'utilisateur dans les conditions empêchant leur développement et, pour la préparation de l'application, on supprime chez l'utilisateur les conditions empêchant le développement et on génère des conditions d'incubation, de telle sorte que les larves éclosent et se développent pour atteindre le stade larvaire sécrétant les sécrétions pour l'application.

4. Procédé selon l'une des revendications précédentes,
**caractérisé en ce que**
les oeufs sont inclus de façon stérile dans le biotope.

5. Procédé selon la revendication 1 ou 2,
**caractérisé en ce que**
les conditions empêchant le développement plus avant des oeufs comprennent une réfrigération et/ou une dessiccation et/ou une évacuation et/ou une atmosphère de gaz inertes et/ou des inhibiteurs chimiques ou biochimiques individuellement ou en combinaison.

6. Procédé selon l'une des revendications précédentes,
**caractérisé en ce que**
les oeufs sont soumis, dans un récipient d'incubation, aux conditions d'incubation, les oeufs dans le récipient d'application étant inclus dans le récipient d'incubation.

7. Dispositif pour la culture de larves de mouches (asticots), comportant un récipient pour accueillir les oeufs des mouches dans des conditions empêchant le développement plus avant des oeufs et un récipient d'incubation, dans lequel les conditions d'incubation pour les oeufs sont maintenues ou générées,
**caractérisé en ce que**
pour recueillir les sécrétions des larves de mouches pour l'application thérapeutique, il est prévu un récipient d'application, dans lequel les oeufs sont placés et maintenus sous des concluons d'incubation, la paroi du récipient d'application étant perméable aux liquides, mais étant imperméable aux larves écloses.

8. Dispositif selon la revendication 7,
**caractérisé en ce que**
le récipient d'application présente une paroi flexible en forme de toile.

9. Dispositif selon la revendication 8,
**caractérisé en ce que**
le récipient d'application est inclus dans le récipient d'incubation.

10. Dispositif selon l'une des revendications 7 à 9,
**caractérisé en ce que**
le récipient d'incubation est un sac en matière plastique.

11. Dispositif selon l'une des revendications 7 à 10,
**caractérisé en ce qu'**
un ou plusieurs rêcipient(s) d'incubation sont logés avec les oeufs inclus dans un récipient d'application dans un récipient dans lequel les conditions empêchant le développement sont maintenues, et dans les récipients d'incubation, on peut tout d'abord générer et maintenir les conditions empêchant le développement puis, au besoin, les transformer en conditions d'incubation et de nutrition.

12. Dispositif selon l'une des revendications 7 à 11,
**caractérisé en ce que** de l'air et/ou une solution nutritive et/ou des composants chimiques/biochimiques/microbiologiques peuvent être introduits à travers la paroi du récipient d'incubation, en particulier au moyen d'une seringue.

13. Dispositif selon l'une des revendications 7 à 11,
**caractérisé en ce que**
de l'air et/ou une solution nutritive et/ou des composants chimiques/biochimiques/microbiologiques contenues dans des réservoirs de stockage fermés qui peuvent être détruits de l'extérieur, sont inclus dans le récipient d'incubation ou placés contre celui-ci.
